# EUROPÄISCHE PATENTSCHRIFT

(11) **EP 0 366 604 B1**
(45) Veröffentlichungstag und Bekanntmachung des Hinweises auf die Patenterteilung: **31.03.1993**
(21) Anmeldenummer: 89810747.9
(22) Anmeldetag: 02.10.1989
(51) Int. Cl.: A61F 2/36

(54) **Schaft für eine Femurkopfprothese**
Stem for a femoral head prosthesis
Tige pour prothèse de tête de fémur

(30) Priorität: 27.10.1988 CH 4001/88
(43) Veröffentlichungstag der Anmeldung: 02.05.1990
(73) Patentinhaber: GEBRÜDER SULZER AKTIENGESELLSCHAFT, CH-8401 Winterthur (CH)
(72) Erfinder: Koch, Rudolf, CH-8267 Berlingen (CH); Frey, Otto, Dr., CH-8400 Winterthur (CH)

(56) Entgegenhaltungen:
- EP-A- 0 190 446
- EP-A- 0 257 222
- DE-A- 3 406 358
- DE-A- 3 536 895
- FR-A- 2 290 881
- FR-A- 2 610 823
- GB-A- 2 070 939
- US-A- 4 516 277

## Beschreibung

Die Erfindung betrifft einen Schaft für eine Femurkopfprothese zur prothetischen Versorgung von Hüftgelenken gemäß dem Oberbegriff des Anspruchs 1. Ein derartiger Schaft ist aus der FR-A-2290 881 bekannt.

Aus der DE-A-31 13 898 ist ein Schaft bekannt, der mindestens über einen Teil seiner Länge hohl ausgebildet ist, wobei die Schaftwand im hohlen Schaftbereich aus einer Vielzahl schmaler Stege besteht; zwischen diesen sind Längsschlitze angeordnet. Der Innenhohlraum dieses Schaftes, der bis zum Prothesenhals in Knochengewebe des Femurs eingebettet ist, ist durch über die Höhe des Hohlraumes im Abstand verteilte, mit Durchbrüchen versehene und als Dübbel bezeichnete Elemente in mehrere Teilräume unterteilt, die zur Unterstützung der Knochenbildung mit Knochensubstanz gefüllt werden. Weiterhin ist eine die Knochenbildung anregende Elektrostimulation vorgesehen, die zusammen mit der eingefüllten Knochensubstanz die Knochenbildung fördern soll, wobei das Knochenwachstum von dem den Schaft peripher umgebenden Knochen her um die Stege herum erfolgen soll. Die Durchbrüche in den Dübeln dienen dabei als Durchführungen für ein oder mehrere Kabel, die die in den Teilräumen angeordneten Kathoden der Elektrostimulation mit einem elektrischen Potential versorgen.

In Fällen von Tumoren oder Reoperationen sind jedoch sehr häufig Hüftgelenke prothetisch zu versorgen, bei denen den Prothesenschaft umhüllende Knochensubstanz zumindest im proximalen Bereich nicht mehr oder nur noch teilweise vorhanden ist. Derartige Prothesen können daher nur in ihrem distalen Bereich auf oder in dem Knochen verankert werden. Der zunächst "knochenfreie" proximale Bereich des Schaftes kann dabei bei solchen Prothesen - konstruktiv ähnlich der eingangs diskutierten bekannten Konstruktion - als von einzelnen Stegen umgebener, durch Stützelemente unterteilter Hohlraum ausgeführt sein, in den, wie bei dem bekannten Schaft, Knochensubstanz eingebracht wird. Die Versorgung und Revaskularisierung dieser Knochensubstanz vom distal gelegenen "Stumpf" des Femurs her durch zunächst an sie anwachsendes und sie dann durchsetzendes Gewebe ist jedoch bei der bekannten Konstruktion durch die praktisch eine geschlossene Barriere bildenden Dübel behindert, wenn nicht gar verunmöglicht, da die Durchbrüche der Dübel von den Kabeln der Elektrstimulation "verschlossen" sind.

Aufgabe der Erfindung ist es daher, bei Reoperations- oder Tumorprothesen der genannten Art eine Revaskularisierung der in den Hohlraum des Schaftes eingebrachten Knochensubstanz von dem stumpfartigen Knochenende des Femurs her zu ermöglichen.

Diese Aufgabe wird mit der Erfindung durch die Merkmale des Kennzeichnenden Teils des Anspruchs 1 gelöst.

Durch die offenen Durchbrüche in den Stützelementen kann aus dem Femurstumpf heraus lebendes Gewebe weitgehend unbehindert in die mit Knochensubstanz gefüllten Hohlräume einwachsen und diese Substanz weitgehend revaskularisieren.

Mit Vorteil können dabei die Durchbrüche und/oder die Längsschlitze zwischen den Stegen mindestens teilweise mit Drahnetzen abgedeckt sein, um ein Durchfallen von Knochensubstanz durch diese Oeffnungen zu verhindern; die Maschenweite der Drahtnetze wird dabei so gewählt, dass sie ein ungehindertes Knochenwachstum durch die Maschen hindurch erlaubt.

Eine bevorzugte Form der Verankerung des Schaftes auf einem Femurstumpf besteht darin, den Schaft auf den Stumpf aufzuschieben und anschliessend mit diesem fest zu verbinden. Dafür ist es zweckmässig, wenn distal des letzten Stützelementes ein nach distal offener Hohlraum vorhanden ist, der durch die aufspreizbar, zungenartige Stege begrenzt ist, wobei eine Befestigung zusätzlich durch mindestens einen distal des letzten Stützelementes angeordneten Klemmring und/oder mit Hilfe von durch Bohrungen der Stege in den Stumpf einschraubbare Knochenschrauben erfolgen kann.

Im folgenden wird die Erfindung anhand von Ausführungsbeispielen im Zusammenhang mit der Zeichnung näher erläutert.
- Fig. 1: gibt schematisch eine Seitenansicht einer ersten Ausführungsform des neuen Schaftes wieder;
- Fig. 2: ist der Schnitt II-II von Fig. 1;
- Fig. 3: zeigt das distale Ende einer zweiten Ausführungsform des Schaftes.

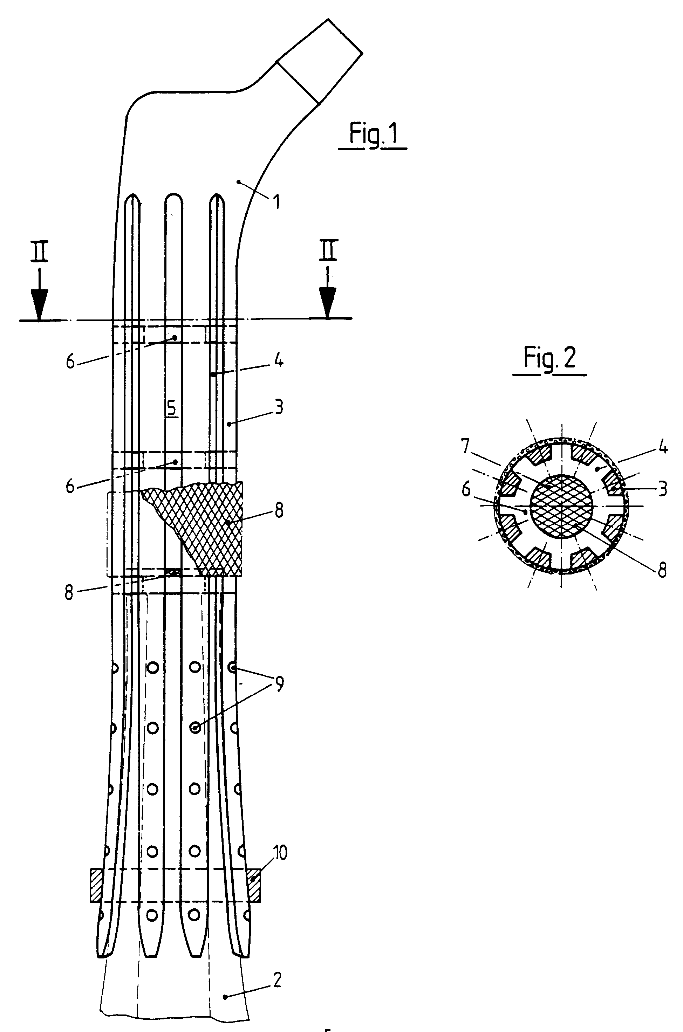

Der als Geradschaft ausgebildete Schaft 1 einer Tumor- oder Reoperationsprothese nach Fig. 1 ist auf einen operativ entsprechend vorbereiteten Femurstumpf 2 aufgesetzt. Er ist daher an seinem einen Ende nach aussen offen und besteht aus einer Anzahl einzelner Stege 3, zwischen denen Längsschlitze 4 vorhanden sind.

Die Stege 3 umschliessen dabei einen Hohlraum 5 im Innern des Schaftes 1. Um die Stabilität des Schaftes 1 zu erhöhen, sind mit Abstand über die Höhe des Hohlraumes 5 scheibenförmige Stützelemente 6 verteilt, die mit den Stegen verbunden sind.

Wie bereits erwähnt, wird der Hohlraum 5 während der Implantation der Prothese mit Knochensubstanz gefüllt. Zu deren Revaskularisierung vom Knochenstumpf 2 her, der bis nahe an das distal letzte Stützelement 6 reicht, sind die Stützelemente 6 mit Durchbrüchen 7 (Fig. 2) versehen, die ein Wachsen der Knochensubstanz in axialer Richtung ermöglichen.

Um das Füllen des Hohlraumes 5 mit Knochenmaterial zu erleichtern, können die Durchbrüche 7 und/oder die Schlitze 4 mit ein- oder mehrlagigen Drahtnetzen 8 mindestens teilweise abgedeckt sein; deren Maschenweiten sind so gewählt, dass ein Knochenwachstum durch die Maschen hindurch ungehindert erfolgen kann, die in den Hohlraum 5 eingefüllten Knochenspäne jedoch zurückgehalten werden. Die die Längsschlitze 4 abdeckenden Drahtnetze 8 können dabei sowohl aussen als auch innen im Hohlraum 5 angeordnet sein. Die bevorzugten Maschenweiten der Netze 8 betragen z.B. 1 bis 1,4 mm.

Im Bereich ausserhalb des letzten Stützelementes 6 sind die Stege 3 bei dem Ausführungsbeispiel nach Fig. 1 aufspreizbar, so dass sie auf den Femurstumpf einen gewissen Druck ausüben. Ihre Verankerung auf dem Knochen kann darüberhinaus zusätzlich durch nicht gezeigte Knochenschrauben, die durch Bohrungen 9 in den Stumpf 2 eingeschraubt werden können, erhöht werden; eine weitere Möglichkeit für eine Verbesserung der Fixierung des Schaftes 1 auf dem Knochen 2 besteht in mindestens einem Klemmring 10, der den Bereich ausserhalb des letzten Stützelementes 6 der Stege 3 umgibt und mit den aufgespreizten Stegen 3 durch Verschieben in distaler Richtung verkeilt werden kann.

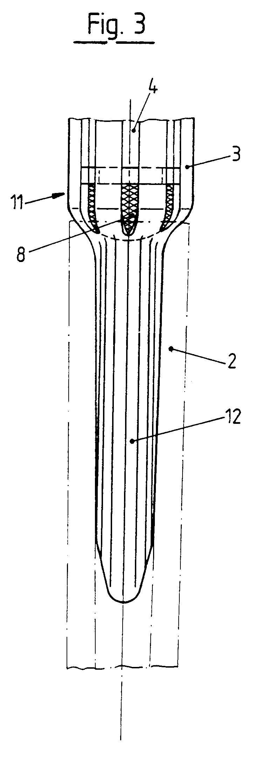

Die zweite Ausführungsform der Erfindung nach Fig. 3 unterscheidet sich von der ersten lediglich in der Art der Verankerung der Prothese im Femurstumpf 2. Dieser ist in Fig. 3 nur rein schematisch angedeutet.

Die Verankerung erfolgt in diesem Fall im Femurstumpf 2 auf konventionelle Weise durch Einsetzen und Einschlagen des Schaftes 11 in einen operativ vorbereiteten Hohlraum im Knochen.

Bei dem Schaft 11 sind daher die Stege 3 ausserhalb des letzten Stützelementes 6 zu einem kompakten distalen Schaftbereich 12 zusammengeführt. Dieser, sich zum freien Ende hin leicht konisch verjüngende Schaftbereich 12 wird in üblicher Weise zementfrei im Knochen oder in einem Knochenzementbett verankert. In bekannter Weise kann er mit einer das An- und Einwachsen von Gewebe oder die Verbindung mit dem Knochenzement fördernde Oberflächenstruktur haben.

Die Versorgung der Knochensubstanz im Innern des Schafthohlraumes erfolgt hier durch vom Stumpfende her aussen in und durch die Schlitze 4 wachsendes Knochengewebe, das zumindest bis zu einer gewissen Höhe auch im Hohlraum weiterwächst und das dortige Knochenmaterial mindestens teilweise revaskularisiert.

## Patentansprüche

1. Schaft (1) für eine Femurkopfprothese zur prothetischen Versorgung von Hüftgelenken, bei denen den Schaft (1) umhüllende Knochensubstanz im proximalen Bereich allenfalls noch teilweise vorhanden ist, so dass die Schaftverankerung ausschliesslich im distalen Bereich erfolgen muss, welcher Schaft (1) mindestens über einen Teil seiner Länge hohl ausgebildet ist, dadurch gekennzeichnet, dass die Schaftwand im hohlen Schaftbereich (5) aus einer Vielzahl schmaler Stege (3) besteht, zwischen denen Längsschlitze (4) angeordnet sind, wobei mit Abstand über die Höhe des Hohlraumes (5) verteilte, scheibenartige Stützelemente (6) die Stege (4) relativ zueinander stabilisieren, und wobei die Stützelemente (6) in Längsrichtung des Schaftes (1) mit Durchbrüchen (7) versehen sind, die im hohlen Schaftbereich (5) ein Knochenwachstum in axialer Richtung des Schaftes (1) zulassen.

2. Schaft nach Anspruch 1, dadurch gekennzeichnet, dass distal des letzten Stützelementes (6) ein nach distal offener Hohlraum vorhanden ist, der durch aufspreizbare, zungenartige Stege (3) begrenzt ist.

3. Schaft nach Anspruch 2, dadurch gekennzeichnet, dass die aufspreizbaren Stege (3) distal des letzten Stützelementes (6) von mindestens einem axial verschiebbaren Klemmring (10) umschlossen sind.

4. Schaft nach Anspruch 2 oder 3, dadurch gekennzeichnet, dass die Stege (3) im aufspreizbaren Bereich mit Bohrungen (9) versehen sind.

5. Schaft nach einem der Ansprüche 1 bis 4, dadurch gekennzeichnet, dass die Durchbrüche (7) in den Stützelementen (6) mit einem Drahtnetz (8) belegt sind, dessen Maschenweite ein ungehindertes Knochenwachstum durch die Maschen hindurch erlauben.

6. Schaft nach einem der Ansprüche 1 bis 5, dadurch gekennzeichnet, dass die Längsschlitze (4) zwischen den Stegen (3) mindestens teilweise von einem Drahtnetz (8) abgedeckt sind.

## Claims

1. A stem (1) for a femur head prosthesis for supplying replacement for hip joints in which the bone substance surrounding the stem (1) is present in only a part, at most, of the proximal region, so that the stem has to be anchored exclusively in the distal region, the stem (1) being hollow along at least part of its length, characterised in that the stem wall in the hollow region (5) comprises a number of narrow webs (3) with slots (4) in between, and disc-like supporting elements (6) are vertically spaced along the cavity (5) and stabilise the webs (4) relative to one another, and the supporting elements (6), in the longitudinal direction of the stem (1), are formed with apertures which enable bone to grow in the hollow region (5) in the axial direction of the stem (1).

2. A stem according to claim 1, characterised in that a distal cavity is provided distally of the last supporting element (6) and is bounded by expandible tongue-like webs (3).

3. A stem according to claim 2, characterised in that the expandible webs (3), distally of the last supporting element (6), are surrounded by at least one axially movable clamping ring (10).

4. A stem according to claim 2 or 3, characterised in that the webs (3) are formed with bores (9) in the expandible region.

5. A stem according to any of claims 1 to 4, characterised in that the apertures (7) in the supporting elements (6) are covered by a wire mesh (8) having an width which enables bone to grow through the meshes unhindered.

6. A stem according to any of claims 1 to 5, characterised in that the slots (4) between the webs (3) are at least partly covered by a wire mesh (8).

## Revendications

1. Tige (1) d'une prothèse de tête de fémur pour la restauration d'articulations de la hanche par prothèse dans lesquelles la substance osseuse entourant la tige (1) existe tout au moins encore partiellement dans la région proximale, de sorte qu'il faut que l'ancrage de la tige soit effectué exclusivement dans la région distale, ladite tige (1) étant creuse au moins sur une partie de sa longueur, caractérisée en ce que la paroi de la région creuse (5) de la tige se compose de multiples lames étroites (3) séparées par des fentes longitudinales (4), des éléments de soutien discoïdaux (6) répartis à distance les uns des autres sur la hauteur de la cavité (5) stabilisant les lames (3) les unes par rapport aux autres et les éléments de soutien (6) comportant dans la direction de la longueur de la tige (1) des traversées (7) qui autorisent une croissance d'os dans la direction axiale de la tige (1) à l'intérieur de la région creuse (5) de la tige.

2. Tige selon la revendication 1, caractérisée en ce qu'elle comprend une cavité ouverte vers le côté distal sur le côté distal du dernier élément de soutien (6), cette cavité étant délimitée par des lames (3) en forme de languettes qui peuvent être écartées.

3. Tige selon la revendication 2, caractérisée en ce que les lames (3) pouvant s'écarter sont entourées sur le côté distal du dernier élément de soutien (6) par au moins un anneau de serrage (10) déplaçable axialement.

4. Tige selon la revendication 2 ou 3, caractérisée en ce que les lames (3) comportent des trous (9) dans la région dans laquelle elles peuvent être écartées.

5. Tige selon l'une des revendications 1 à 4, caractérisée en ce que les traversées (7) des éléments de soutien (6) sont garnies d'une toile métallique (8) dont l'ouverture des mailles permet une libre croissance de l'os à travers les mailles.

6. Tige selon l'une des revendications 1 à 5, caractérisée en ce que les fentes longitudinales (4) séparant les lames (3) sont recouvertes au moins partiellement d'une toile métallique (8).
